(19) Europäisches Patentamt
European Patent Office
Office européen des brevets

(11) Publication number: **0 462 522 A1**

(12) **EUROPEAN PATENT APPLICATION**

(21) Application number: **91109785.5**

(22) Date of filing: **14.06.91**

(51) Int. Cl.[5]: **C07D 403/04**, A61K 31/55, //(C07D403/04,243:00,207:00)

(30) Priority: **18.06.90 US 539500**

(43) Date of publication of application: **27.12.91 Bulletin 91/52**

(84) Designated Contracting States: **AT BE CH DE DK ES FR GB GR IT LI LU NL SE**

(71) Applicant: **F. HOFFMANN-LA ROCHE AG**
**Postfach 3255**
**CH-4002 Basel(CH)**

(72) Inventor: **Bellemin, Anne Roberte**
**27 Hillcrest Avenue**
**Cranford, N.J. 07016(US)**
Inventor: **Earley, James Valentine**
**40 Daniel Drive**
**Cedar Grove, N.J. 07009(US)**
Inventor: **Hsu, Ming-Chu**
**445 East 86th Street**
**New York, N.Y. 10028(US)**
Inventor: **Tam, Steve Yik-Kai**
**13 Evergreen Road**
**West Caldwell, N.J. 07006(US)**

(74) Representative: **Lederer, Franz, Dr. et al**
**Lederer, Keller & Riederer, Patentanwälte,**
**Lucile-Grahn-Strasse 22**
**W-8000 München 80(DE)**

(54) **Aminobenzodiazepines.**

(57) Aminobenzodiazepines of formula

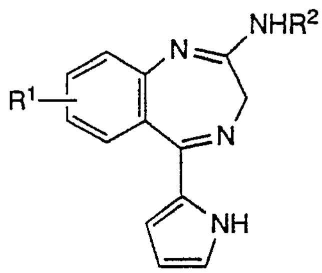

I

wherein $R^1$ is H, $NO_2$, halogen, $CF_3$, lower alkyl, OH, lower alkoxy or CN, and $R^2$ is H or $CH_3$,
and pharmaceutically acceptable salts thereof possess antiviral activity especially for the treatment or prophylaxis of viral infections, particularly of retroviral infections, such as HIV 1 and/or HIV 2 infections, or for protecting cells against such infections.

They can be prepared from corresponding benzodiazepinethiones.

EP 0 462 522 A1

Figure 1

Anti–HIV Activity of Compound A in CEM Cells Infected with HIV-1

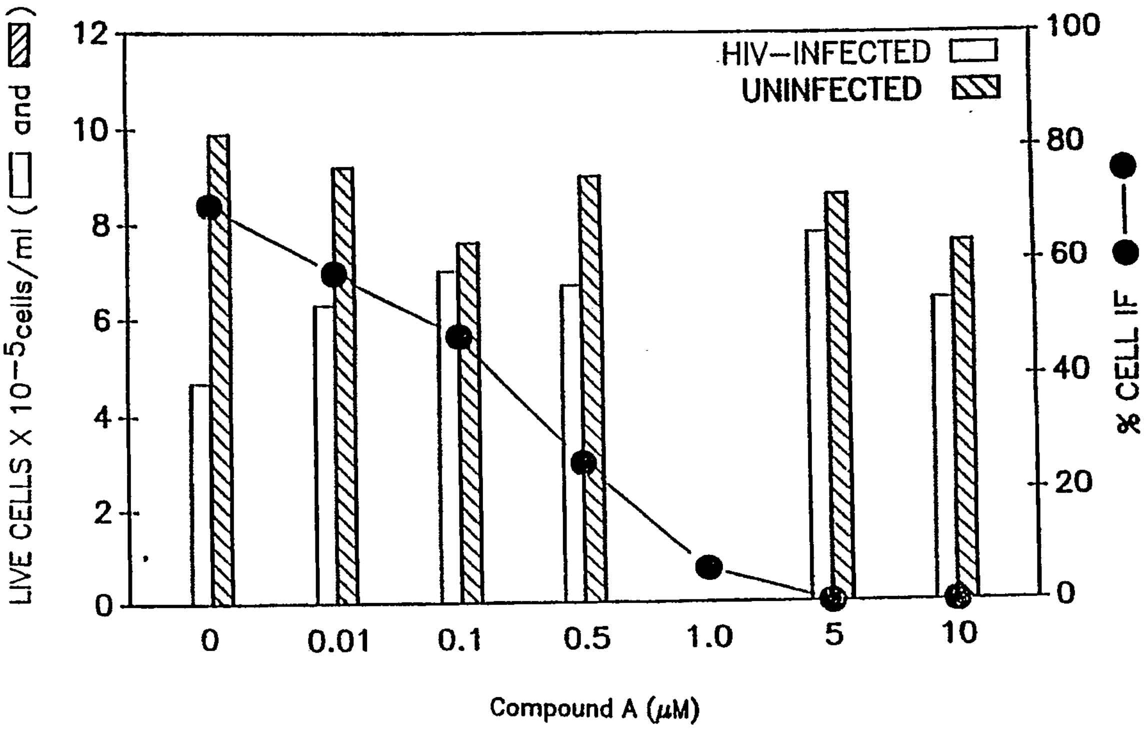

The present invention relates to aminobenzodiazepines of formula

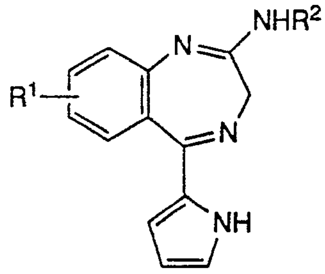

I

wherein $R^1$ is H, $NO_2$, halogen, $CF_3$, lower alkyl, OH, lower alkoxy or CN, and $R^2$ is H or $CH_3$, and pharmaceutically acceptable salts thereof.

Objects of the present invention are the above compounds per se and for use as a therapeutically active agent, especially for the treatment or prophylaxis of viral infections, particularly of retroviral infections, such as HIV 1 and/or HIV 2 infections, or for protecting cells against such infections;

further a process for the manufacture of these compounds and medicaments containing one of such compounds and, optionally, a second antiviral agent, especially a reverse transcriptase inhibitor, such as ddC, AZT, a HIV-protease inhibitor, $\alpha$-,$\beta$- and/or $\gamma$-interferon, interleukin-2 and/or GM-CSF, and

the use of these compounds for the manufacture of medicaments especially for the treatment or prophylaxis of viral infections, particularly of retroviral infections, such as HIV 1 and/or HIV 2 infections, or for protecting cells against such infections.

As used therein, the term "lower" refers to straight or branched hydrocarbon chains with up to 7 C atoms, such as methyl, ethyl, propyl and isopropyl.

Pharmaceutically acceptable salts may be those with organic acids, e.g. lactic, acetic, malic or p-toluenesulfonic acid; or salts with mineral acids, such as hydrochloric or sulfuric acid.

The above compounds can exist in stereoisomeric or tautomeric forms, all being included within the scope of the invention.

Preferred compounds of the invention are those wherein $R^1$ is Cl, especially 7-chloro-N-methyl-5-(1H-pyrrol-2-yl)-3H-1,4-benzodiazepin-2-amine.

The compounds of formula I can be prepared by reacting a benzodiazepinethione of formula

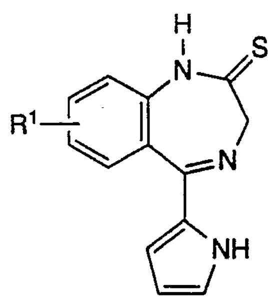

II

wherein $R^1$ is as in above,

or the corresponding amide wherein O stands for S, with a compound of formula $R^2$-$NH_2$. The reaction can be performed in a conventional manner, e.g. by reacting the benzodiazepinethione in THF and methanol at -78°C with ammonia or by reacting the corresponding amide with an amine $R^2$-$NH_2$ in an inert solvent, such as THF, toluene or dioxane, in the presence of a Lewis acid, such as $TiCl_4$ or $SnCl_4$. The benzodiazepinethiones II can be prepared by reacting the corresponding amide with phosphorous pentasulfide or with 2,4-bis(4-methoxyphenyl)-1,3-dithio-2,4-diphosphetane-2,4-disulfide(Lawessen's reagent), e.g. in THF, toluene, pyridine or dioxane, preferably at 50-100°C.

The compounds I and their salts have useful antiviral, especially antiretroviral activity, particularly against HIV, the virus implicated in the development of AIDS and related diseases such as ARC (AIDS related complex). These compounds also inhibit HIV replication by inhibiting such important HIV viral functions as TAT (transactivating transcriptional) activity.

The compounds I were tested for the inhibition of HIV-cytopathic effect and the reduction of cell-associated viral antigens in an experiment run as follows:

High titer virus stocks (HIV-1 NIT strain) were grown in $CD_4$ + CR10 cells in RMPI-1640 media (Gibco Laboratories) supplemented with 10% fetal calf serum and 0.1 mg/ml Gentamicin. The collected media were filtered and the virus isolates were concentrated 100 fold and stored at -80 °C. $CD_4$ + CEM cells, propagated in the same medium, were incubated for 60 minutes at 37 °C with diluted stock virus at multiplicity of infection (MOI) equal to 1. Cells were washed with phosphate buffered saline and resuspended in the medium at 2 x $10^5$ cells/ml. Various quantities of the test compounds in DMSO were added. Four days after infection, the number of live cells were counted by trypan blue exclusion (Proc. Natl. Acad. Sci. 83, 1986, 1911). At the same time, aliquots of cells were fixed with acetone and stained with antibodies from AIDS patients, followed by a second staining with fluorescein-conjugated goat anti-human IgG (Cappel). Cells stained with the fluorescent antibody were counted using a fluorescence microscope and the results were expressed as percentage of the total number of cells counted (J. Med. Virol. 19, 1986, 325).

The results expressed in $ID_{50}$ ($\mu$M), i.e. the concentration of the test compound which reduces the number of immunofluorescence positive cells by 50%, amounts to 0.5 to 1 $\mu$M for the product of Example 1 (Compound A) and to 1 $\mu$M for the product of Example 2 (Compound B).

For cytotoxicity testing, CEM cells were treated with a compound of formula I at similar concentrations and the toxicity of the compound was measured by the live-cell count.

Figures 1 and 2 depict the anti-HIV activity of Compound A. Figure 1 shows the survival rate of HIV-1-infected CEM-cells treated with Compound A. Figure 1 also shows that treatment of cells with increasing amounts of Compound A results in the decline in the percentage of cells that are immunofluorescence-positive following staining with HIV antibodies. Figure 2 provides a comparison of anti-HIV activity, as measured by cell survival and $p^{24}$ antigen and viral RNA amount of Compound A versus AZT and ddC.

The Compounds A and B were also tested for anti-HIV-TAT activity in an assay comprising the following steps:

(a) putting both the expression of the Secreted Alkaline Phosphatase (SeAP) gene and the viral transactivator TAT gene under the control of the HIV promoter LTR responsive to the action of the HIV transactivator TAT;

(b) transfecting cultured mammalian cells with plasmids which contain the gene constructs of (a) above and cause cellular production of the transactivating factor TAT and SeAP;

(c) adding the agent to be tested, here Compounds A and B; and determining the amount of SeAP produced, by measuring SeAP enzymatic activity, whereby inhibition of SeAP production correlates with the anti-TAT inhibition activity. In this assay, the inhibition of SeAP positively correlates with anti-TAT activity. The greater the ability of an agent to inhibit SeAP, the greater is its anti-TAT activity.

Specifically, with respect to the results reported below, the anti-HIV-TAT assay was run as follows:

At 24 hours post transfection 1, 10, 25 and 50 $\mu$M of a test compound of formula I was added to the culture media of COS cells transfected with two plasmids, one containing the reporter gene which codes for SeAP under control of HIV-LTR, and the other containing the HIV-TAT gene also under control of HIV-LTR. The alkaline phosphatase activity of the media was assayed 48 hours after addition of test compound with a colorimetric assay using p-nitrophenylphosphate as the substrate. The anti-TAT activity is measured by the percent inhibition of SeAP gene expression under the control of HIV-LTR versus the percent inhibition of SeAP gene under RSV-LTR, which does not respond to TAT. An inhibition superior to 60% was measured as the average of three independent assays of each test Compound A and B.

These results show that Compounds A and B are specific inhibitors of HIV-TAT-regulated gene expression without non-specific cytotoxic effects.

The specificity of Compounds A and B as TAT inhibitors was demonstrated with a parallel assay in which the SeAP gene expression is put under control of the Rous sarcoma virus (RSV)-LTR which does not respond to TAT. This assay thus eliminates the possibility that Compounds A and B are either general cytotoxic agents or inhibit the activity of SeAP.

The anti-HIV-TAT activities of the test compounds were determined by measuring the amount of alkaline phosphatase in the supernatant media of cultures of cells in which SeAP gene expression was under the control of the HIV LTR promoter. The specific inhibitory activities of the test compounds were calculated according to the formula:

$$100 [(1-A/B) - (1-C/D)]$$

where A and B are the alkaline phosphatase activites produced by HIV-LTR/SeAP in the presence and absence, respectively, of test compound, and C and D are the alkaline phosphatase activities produced by

RSV-LTR/SeAP in the presence and absence, respectively, of test compound. The concentrations tested ranged from 1-50 μM. The results provided are the average of at least three tests. The test compound was added 24 hours after cells were transfected with the plasmids when SeAP specific mRNA and protein were already present and the protein was very stable. Therefore, 100% inhibition would not be observed with this assay procedure. Figure 3 reports the results of the assays for Compound A.

Compound A was also tested for anti-HIV-TAT activity with cell lines that constitutively express the SeAP gene under the control of the HIV-LTR. Cell lines 193C, 191F and 199F were derived from CHO cells (cell line of Chinese hamster ovary origin). Each line is of clonal origin and has the HIV-LTR/SeAP and the HIV-LTR/TAT sequences integrated in the cellular chromosome. One day after cells were plated (approximately 30% confluency), the Compound A in DMSO (0.05% concentration) was added to the culture media at desired concentrations. The cells were washed and Compound A was replenished one day later. SeAP activity was assayed two days after the second addition of test compound. The results of the cytotoxicity measurement given in Figure 4 show that while the Compound A significantly inhibits SeAP expression under the control of the HIV-LTR promoter (demonstrated by the top set of lines), it has no cytotoxic effect on the cells (bottom set of dashed lines).

With respect to human patients infected with HIV, and patients with symptomatic or asymptomatic HIV infections, an antivirally-effective amount of a compound of formula I or a salt thereof is in the range of from about 0.1 to 10 mg/kg, preferably from about 0.3 to 5 mg/kg, more preferably from about 1 to 3 mg/kg body weight per day. This dosage may be administered parenterally or orally in one or more doses at various intervals daily, preferably orally once daily.

The compounds may also be administered with other antiviral and/or biological response modifiers. For example, they may be administered with known RT (reverse transcriptase) inhibitors such as ddC, AZT and ddI or other inhibitors which act against other HIV proteins such as protease, as well as with biological modifiers such as $\alpha$, $\beta$ and/or $\gamma$-interferon, interleukin-2 and/or GM-CSF. For ddC, a range of about 0.005-0.25 mg/kg body weight is virustatic in most patients. The preliminary dose ranges for oral administration are somewhat broader, for example 0.001 to 0.25 mg/kg given in one or more doses at intervals of 2, 4, 6, 8 or 12 hours. Currently 0.01 mg/kg body weight ddC given every 8 hours is preferred. When given in combined therapy, the other anti-HIV compounds may be given at the same time as a compound of the invention or the dosing may be staggered as desired. The two (or more) drugs may also be combined in a composition. Doses of each drug may be less when used in combination than when they are used as a single agent.

It is possible for the compounds of the invention to be administered alone in solution. However, it is preferred that the active ingredients be administered in a pharmaceutical formulation or composition. These formulations comprise at least one active ingredient together with one or more pharmaceutically acceptable carrier and excipient and may optionally include other therapeutic agents, for example a protease inhibitor. These carriers include those suitable for oral, rectal, nasal, topical, buccal, sublingual, vaginal or parenteral (including subcutaneous, intramuscular, intravenous and intradermal) administration.

Examples of compositions of the invention are solutions of the active ingredient(s), e.g. in water or saline; capsules, e.g. soft gelatine capsules; sachets or tablets, each containing a pre-determined amount of the active ingredient, e.g. as granules; solutions or suspensions in an aqueous liquid or in an oil-in-water emulsion or a water-in-oil liquid emulsion. Tablets may include one or more of lactose, microcrystalline cellulose, colloidal silicon dioxide, croscarmellose sodium, magnesium stearate, stearic acid and other excipients, colorants and pharmacologically compatible carriers. Formulations suitable for topical administration include lozenges comprising the active ingredient in a flavor, usually sucrose and acacia or tragacanth; pastilles comprising the active ingredient in an inert basis such as gelatin and glycerin, or sucrose and acacia; and mouthwashes comprising the active ingredient in a suitable liquid carrier. Formulations for rectal administration may be presented as a suppository with a suitable base comprising cocoa butter or a salicylate. Formulations suitable for vaginal administration may be presented as pessaries, tampons, creams, gells, pastes, foams or spray formulas. Formulations suitable for parenteral administration include aqueous and non-aqueous, isotonic sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example ampules and vials, and may be stored in a lyophilized condition requiring only the addition of the sterile liquid carrier, for example water for injections, immediately prior to use. Extemporaneous injection solutions and suspensions may be prepared from sterile powder, granules and tablets of the kind previously described.

Example 1

A solution of 0.5 g of 7-chloro-1,3-dihydro-5-(1H-pyrrol-2-yl)-1,4-benzodiazepin-2-one in 25 ml of THF is cooled in an ice/acetone bath. Methylamine is bubbled through the solution. To this solution, 0.29 ml of $TiCl_4$ are added dropwise with stirring and, after 5 hours at room temperature, additional 0.2 ml of $TiCl_4$ are added. After 18 hours a small piece of ice is added and the mixture is filtered, washed with THF and concentrated. Crystallization from $CH_2Cl_2$/methanol and recrystallization from ethanol gives 0.25 g (48%) of 7-chloro-N-methyl-5-(1H-pyrrol-2-yl)-3H-1,4-benzodiazepin-2-amine, mp 251 °C.

Example 2

a) To a solution of 10 g of 7-chloro-1,3-dihydro-5-(1H-pyrrol-2-yl)-1,4-benzodiazepin-2-one in 400 ml THF, 10 g of $P_4S_{10}$ are added under stirring and the mixture is irradiated with ultrasound at 40 °C. After 2.5 hours, additional 10 g of $P_4S_{10}$ are added to the mixture. The reaction is continued for 2 hours. The mixture is cooled to ambient temperature and filtered. After washing the solid product with $CM_2Cl_2$, 80% of the solvent are removed in vacuo from the combined filtrates. The residual solution is basified with saturated sodium bicarbonate solution. The product which precipitates out is filtered. The filtrate is extracted with $CM_2Cl_2$, followed by concentration in vacuo. The solids are recrystallized in THF/Petroleum ether to give 9.9 g (94% yield) of 7-chloro-1,3-dihydro-5-(1H-pyrrol-2-yl)-1,4-benzodiazepin-2-thione, mp 258-260 °C dec.

b) Ammonia is bubbled through a solution of 0.5 g of the product of a) in 5 ml of THF and 200 ml of methanol, cooled to -78 °C. The reaction is then allowed to warm up to room temperature and stirred for another 12 hours. The solvent is evaporated to give 7-chloro-5-(1H-pyrrol-2-yl)-3H-1,4-benzodiazepin-2-amine in 40% yield, mp 177-179 °C dec.

The following galenical compositions containing a compound I or a salt thereof as active ingredients as defined above, can be prepared in a manner known per se:

Tablet formulation:

| Ingredients | mg/tablet |
| --- | --- |
| Active ingredient | 20 mg |
| Lactose | 180 mg |
| Pregelatinized starch | 15 mg |

Oral liquid formulation:

| Ingredients | mg/formulation |
| --- | --- |
| Active ingredient | 20.0 mg |
| Methylparaben | 20.0 mg |
| Sucrose | q.s. |
| Flavoring agent | q.s. |
| Citrate buffer | q.s. |
| Purified water q.s. | 5.0 ml |

Tablet formulation:

| Ingredients | mg/tablet |
| --- | --- |
| Active ingredient | 20 mg |
| Starch | 40 mg |
| Avicel | 80 mg |
| Lactose | 274 mg |
| Magnesium stearate | 2 mg |
| | 416 mg |

Soft gelatine capsule formulation:

| Ingredients | mg/capsule |
| --- | --- |
| Active ingredient | 20 mg |
| Ethoxylated Fatty acids | 500 mg |
| PEG 4000 | 100 mg |
| Vegetable oils q.s. to | 1.0 ml |

## Claims

1. Aminobenzodiazepines of formula

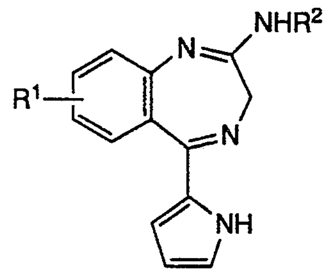

I

wherein $R^1$ is H, $NO_2$, halogen, $CF_3$, lower alkyl, OH, lower alkoxy or CN, and $R^2$ is H or $CH_3$, and pharmaceutically acceptable salts thereof.

2. Compounds as in claim 1, wherein $R^1$ is Cl.

3. 7-Chloro-N-methyl-5-(1H-pyrrol-2-yl)-3H-1,4-benzodiazepin-2-amine.

4. Benzodiazepinethiones of formula

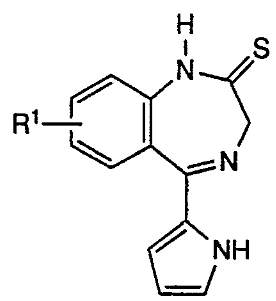

II

wherein $R^1$ is as in claim 1.

5. A compound according to claim 1, 2 or 3 for use as a therapeutically active agent, especially for the treatment or prophylaxis of viral infections, particularly of retroviral infections, such as HIV 1 and/or HIV 2 infections, or for protecting cells against such infections.

6. A process for preparing a compound as in claim 1, which comprises reacting a benzodiazepine of formula II as in claim 4 or the corresponding amide wherein O stands for S, with a compound of formula $R^2$-$NH_2$.

7. A medicament, especially for the treatment or prophylaxis of viral infections, particularly of retroviral infections, such as HIV 1 and/or HIV 2 infections, or for protecting cells against such infections, containing as active pharmaceutical ingredient a compound as in claim 1, 2 or 3 and, optionally, a second antiviral agent, especially a reverse transcriptase inhibitor, such as ddC, AZT, a HIV-protease inhibitor, $\alpha$-,$\beta$- and/or $\gamma$-interferon, interleukin-2 and/or GM-CSF.

8. The use of a compound as in claim 1,2 or 3, for the manufacture of a medicament especially for the treatment or prophylaxis of viral infections, particularly of retroviral infections, such as HIV 1 and/or HIV 2 infections, or for protecting cells against such infections.

**Claims for the following Contracting States: GR**

1. A process for the manufacture of the aminobensodiazepines of formula

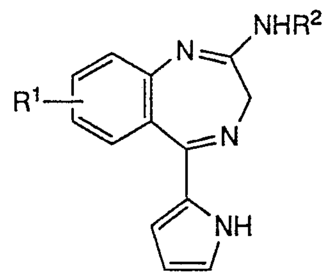

I

wherein $R^1$ is H, $NO_2$, halogen, $CF_3$, lower alkyl, OH, lower alkoxy or CN, and $R^2$ is H or $CH_3$, and pharmaceutically acceptable salts thereof, which process comprises reacting a benzodiazepine of formula

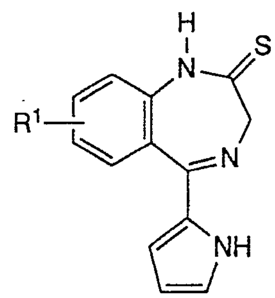

II

or the corresponding amide wherein O stands for S, with a compound of formula $R^2$-$NH_2$.

2. A process as in claim 1, wherein $R^1$ is Cl.

3. A process as in claim 1 or 2, wherein 7-chloro-N-methyl-5-(1H-pyrrol-2-yl)-3H-1,4-benzodiazepin-2-amine is prepared.

4. A process for the manufacture of a medicament, especially for the treatment or prophylaxis of viral infections, particularly of retroviral infections, such as HIV 1 and/or HIV 2 infections, or for protecting cells against such infections, which process comprises bringing a compound of formula I or a salt thereof according to claim 1, 2 or 3, and, optionally, a second antiviral agent, especially a reverse transcriptase inhibitor, such as ddC, AZT, a HIV-protease inhibitor, $\alpha$-,$\beta$- and/or $\gamma$-interferon, interleukin-2 and/or GM-CSF, into a galenical form.

5. The use of a compound as in claim 1, 2 or 3, for the manufacture of a medicament especially for the treatment or prophylaxis of viral infections, particularly of retroviral infections, such as HIV 1 and/or HIV 2 infections, or for protecting cells against such infections.

6. Benzodiazepinethiones of formula

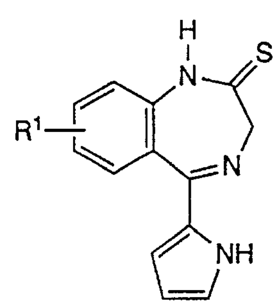

II

wherein $R^1$ is as in claim 1.

**Claims for the following Contracting States: ES**

1. A process for the manufacture of the aminobenzodiazepines of formula

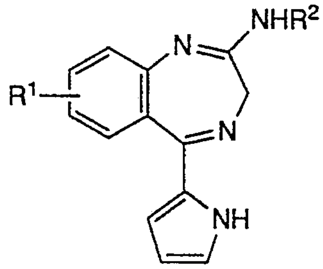

I

wherein $R^1$ is H, $NO_2$, halogen, $CF_3$, lower alkyl, OH, lower alkoxy or CN, and $R^2$ is H or $CH_3$, and pharmaceutically acceptable salts thereof, which process comprises reacting a benzodiazepine of formula

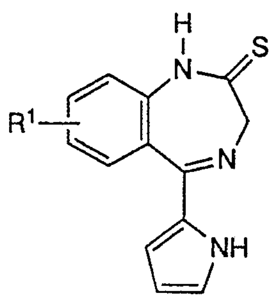

II

or the corresponding amide wherein O stands for S, with a compound of formula $R^2$-$NH_2$.

2. A process as in claim 1, wherein $R^1$ is Cl.

3. A process as in claim 1 or 2, wherein 7-chloro-N-methyl-5-(1H-pyrrol-2-yl)-3H-1,4-benzodiazepin-2-amine is prepared.

4. The use of a compound as in claim 1, 2 or 3, for the manufacture of a medicament especially for the treatment or prophylaxis of viral infections, particularly of retroviral infections, such as HIV 1 and/or HIV 2 infections, or for protecting cells against such infections.

5. The compounds, compositions, processes and uses, whenever described hereinabove, particularly with reference to the Examples.

Figure 1

Anti–HIV Activity of Compound A in CEM Cells Infected with HIV–1

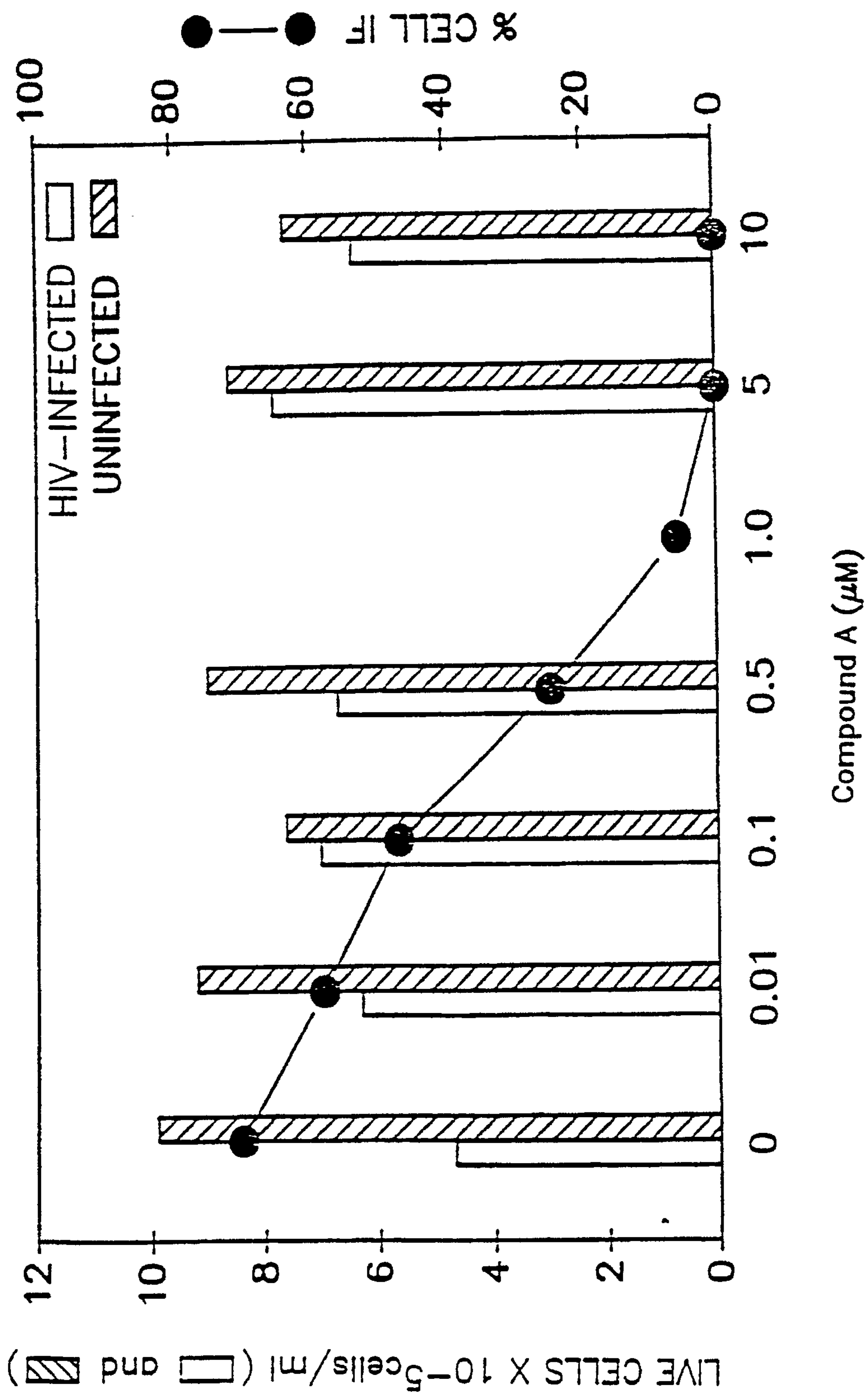

Anti-HIV Activity of Compound A in CEM Cells Infected with HIV-1

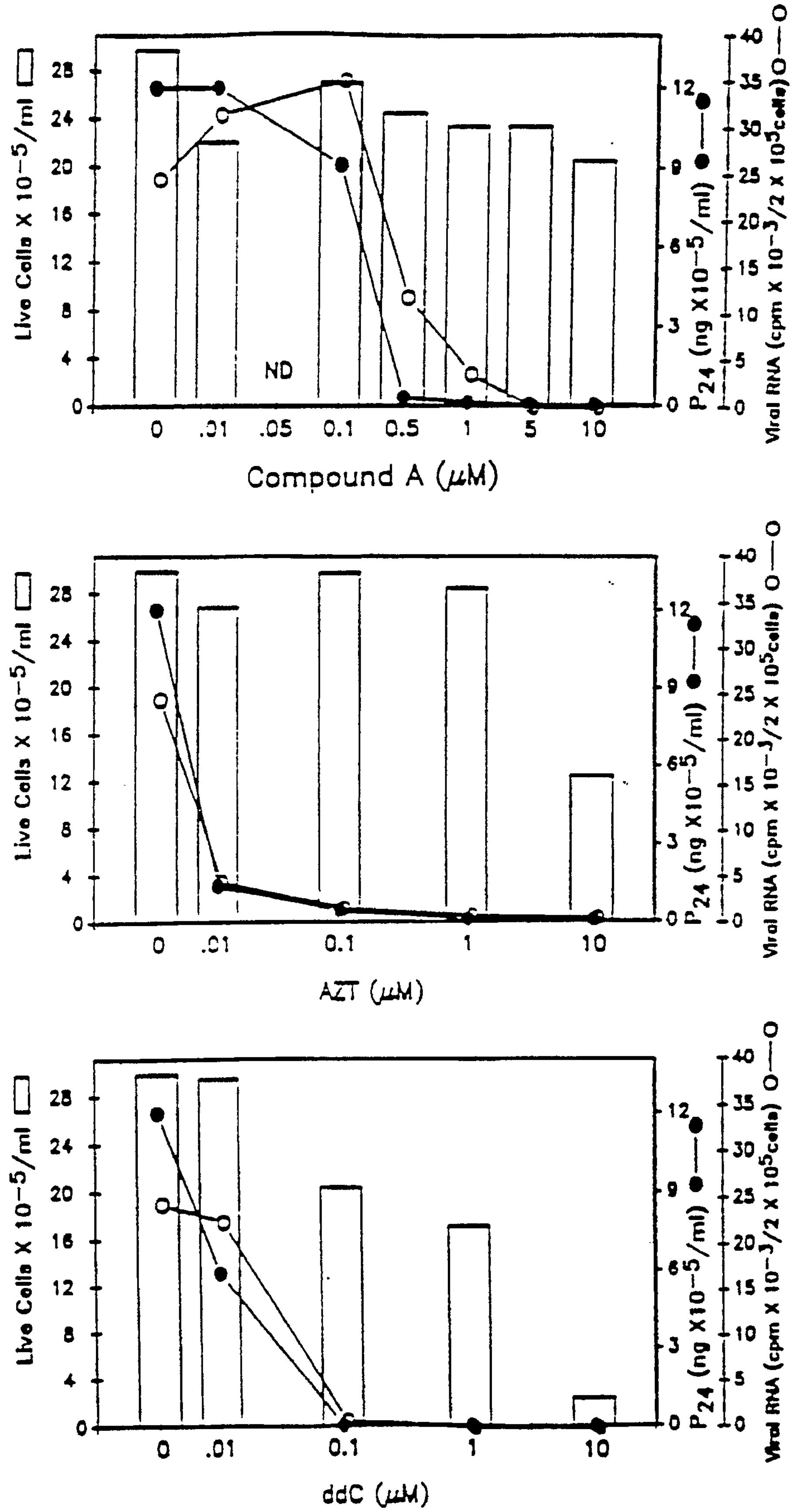

Figure 3

Anti-TAT Activity of Compound A Demonstrated With a Transfection-Based Assay

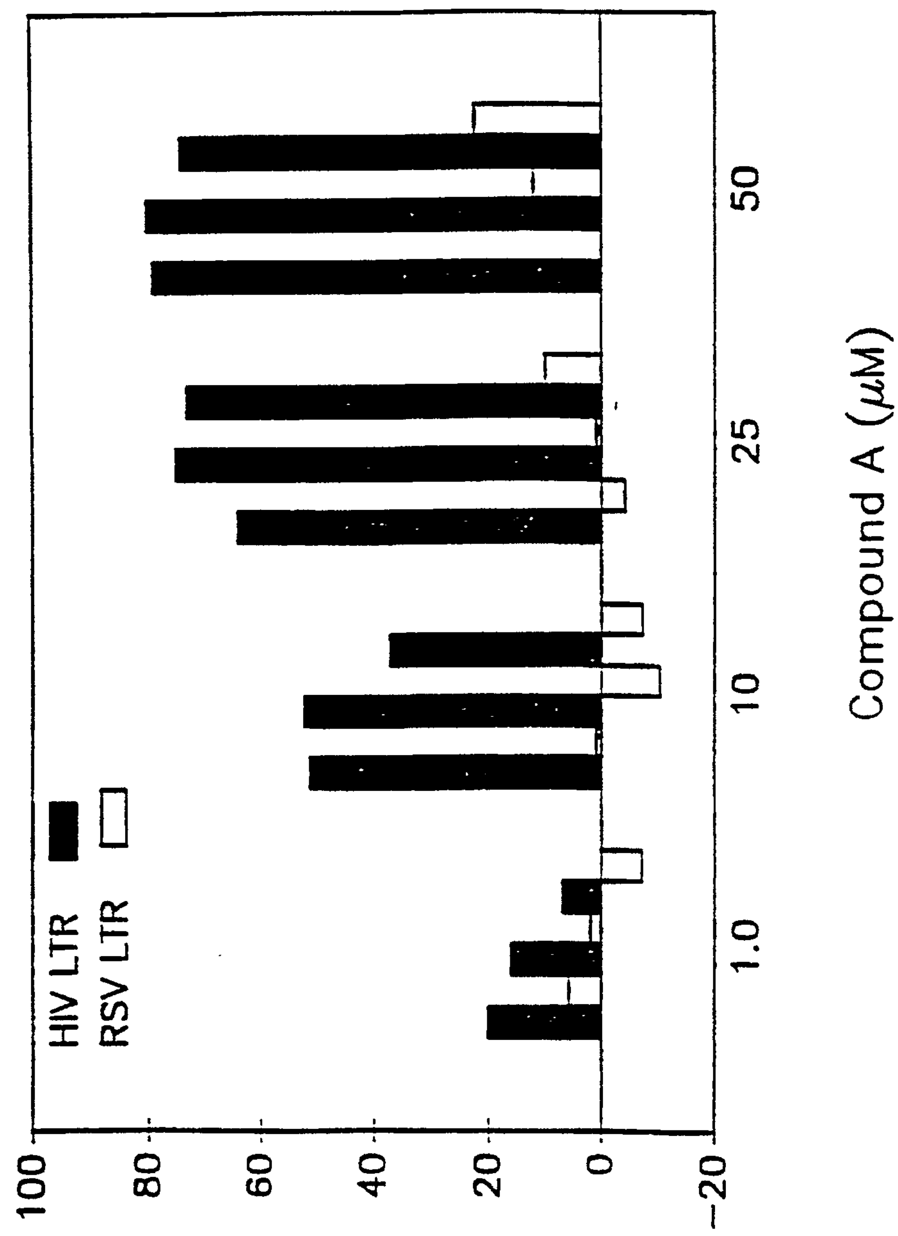

# Figure 4

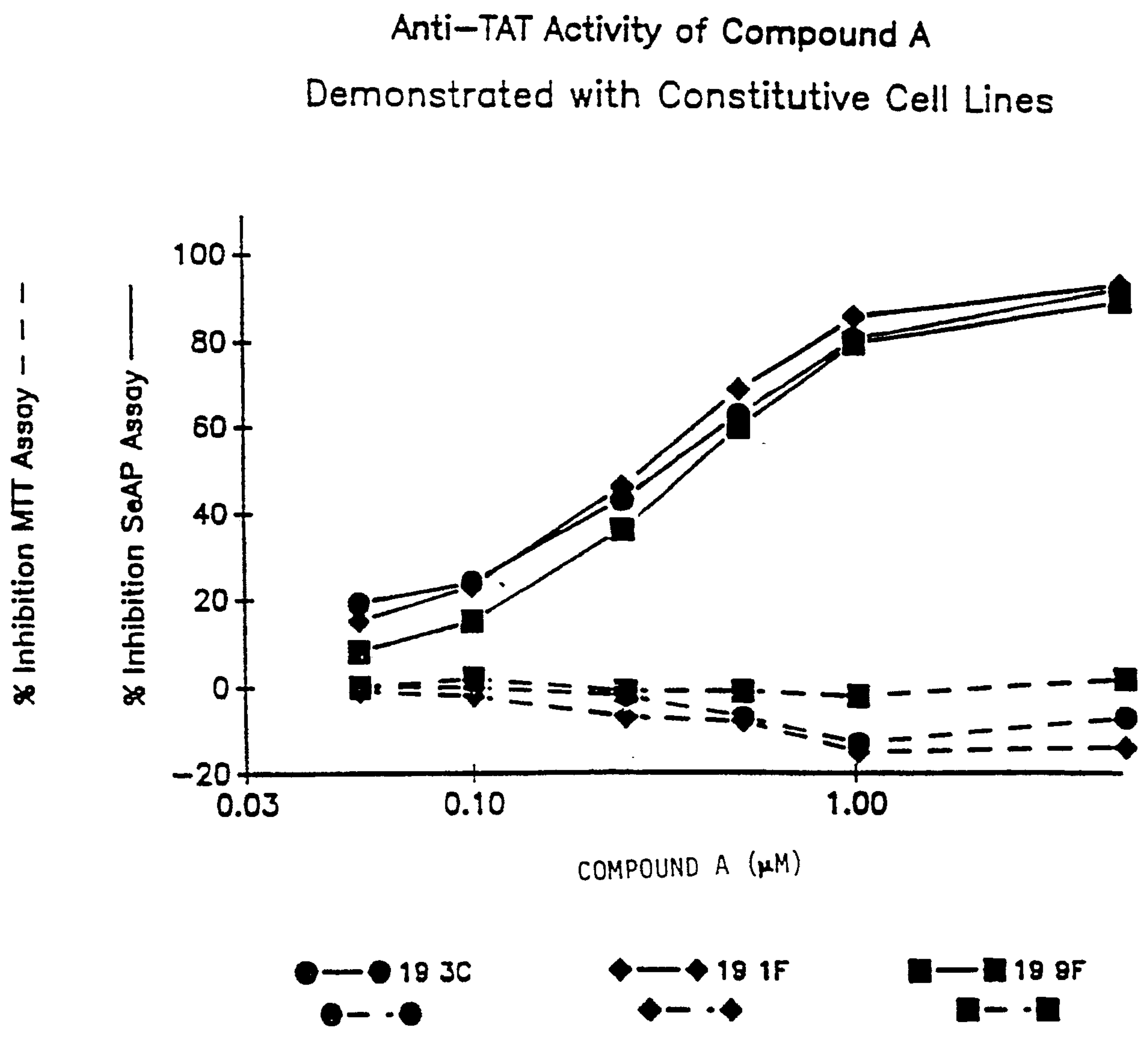
Anti–TAT Activity of Compound A
Demonstrated with Constitutive Cell Lines
% Inhibition MTT Assay - - -
% Inhibition SeAP Assay ——
100
80
60
40
20
0
−20
0.03
0.10
1.00
COMPOUND A (µM)
19 3C
19 1F
19 9F

European Patent Office

# EUROPEAN SEARCH REPORT

Application Number

**EP 91 10 9785**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE-B-2 017 060 (SUMITOMO CHEMICAL CO., LTD.)<br>* column 12, lines 9 - 10 * | 1,4 | C 07 D 403/04<br>A 61 K 31/55 //<br>(C 07 D 403/04<br>C 07 D<br>C 07 D 243:00<br>C 07 D 207:00 ) |
| A | DE-A-1 445 878 (F. HOFFMANN-LA ROCHE AND CO. AG)<br>* examples 6, 7; claim 1 * | 1,2,4,6 | |
| A | DE-A-1 695 225 (F. HOFFMANN-LA ROCHE AND CO. AG)<br>* claim 1 * | 1,4,6 | |
| A,D | PROCEEDINGS OF THE NATIONAL ACADEMY OF SCIENCES OF THE U.S.A., vol. 83, no. 6, March 1986, pages 1911 - 1915; H. MITSUYA et al.: "Inhibition of the in vitro infectivity and cytopathic effect of human T-lymphotrophic virus type III/lymphadenopathy-associated ..."<br>* page 1914, figure 5 * | 7,8 | |

| TECHNICAL FIELDS SEARCHED (Int. Cl.5) |
|---|
| C 07 D 243/00<br>C 07 D 403/00 |

The present search report has been drawn up for all claims

| Place of search | Date of completion of search | Examiner |
|---|---|---|
| Berlin | 29 August 91 | HASS C V F |

CATEGORY OF CITED DOCUMENTS

- X: particularly relevant if taken alone
- Y: particularly relevant if combined with another document of the same catagory
- A: technological background
- O: non-written disclosure
- P: intermediate document
- T: theory or principle underlying the invention
- E: earlier patent document, but published on, or after the filing date
- D: document cited in the application
- L: document cited for other reasons
- &: member of the same patent family, corresponding document